# EUROPEAN PATENT APPLICATION

(11) **EP 4 467 967 A1**
(43) Date of publication of application: **27.11.2024**
(21) Application number: 23740316.7
(22) Date of filing: 12.01.2023
(51) Int. Cl.: G01N 21/65, C12Q 1/06

(54) **METHOD FOR EVALUATING CARDIOCYTES USING RAMAN SCATTERING**

(30) Priority: 17.01.2022 JP 2022005252
(71) Applicant: OSAKA UNIVERSITY, Suita-shi Osaka 565-0871 (JP); NATIONAL INSTITUTE OF ADVANCED INDUSTRIAL SCIENCE AND TECHNOLOGY, Tokyo 100-8921 (JP)
(72) Inventor: FUJITA Katsumasa, Suita-shi, Osaka 565-0871 (JP); BANDO Kazuki, Suita-shi, Osaka 565-0871 (JP); LIU Li, Suita-shi, Osaka 565-0871 (JP); LI Junjun, Suita-shi, Osaka 565-0871 (JP); SAWA Yoshiki, Suita-shi, Osaka 565-0871 (JP); MIYAGAWA Shigeru, Suita-shi, Osaka 565-0871 (JP); NAWA Yasunori, Ikeda-shi, Osaka 563-8577 (JP); FUJITA Satoshi, Ikeda-shi, Osaka 563-8577 (JP)
(74) Representative: Banse & Steglich Patentanwälte PartmbB
(86) International application number: PCT/JP2023/000671
(87) International publication number: WO 2023/136308

(57) **Abstract**

Provided are methods for evaluating differentiation into cardiomyocytes and maturation of cardiomyocytes. Method for evaluating cardiomyocytes using Raman scattering: a Raman spectrum of cardiomyocytes artificially induced to differentiate from pluripotent stem cells is acquired, an intensity of Raman-scattered light for a protein containing at least one of heme b and heme c as a prosthetic group is acquired from the Raman spectrum, and a state of progress of maturation of the cardiomyocytes is evaluated on the basis of the intensity of the Raman-scattered light. Method for evaluating differentiation into cardiomyocytes using Raman scattering: cells which are pluripotent stem cells are artificially induced to differentiate into cardiomyocytes, a Raman spectrum of the cells induced to differentiate is acquired, an intensity of Raman-scattered light for at least one of heme b and heme c is acquired from the Raman spectrum, and a state of progress of differentiation of the cells into cardiomyocytes is evaluated on the basis of the intensity of the Raman-scattered light.

## Description

### Technical Field

The present invention relates to methods for evaluating cardiomyocytes using Raman scattering.

### Background Art

With the progress of studies on iPS cells and other pluripotent stem cells, studies aimed at differentiation of pluripotent stem cells into various organ cells and clinical application thereof are continually conducted. Studies to induce cardiomyocytes from pluripotent stem cells are also active. As an outcome of the studies, a thin sheet of cardiomyocytes can be artificially produced.

In clinical medicine, cardiomyocytes differentiated from pluripotent stem cells as a result of artificial induction are formed into a sheet shape, and then transplanted into an intended patient. This requires establishment of methods for evaluating cardiomyocytes which are being differentiated, cardiomyocytes immediately after differentiation, and cardiomyocytes formed into a sheet shape, as well as reliable quality assurance based on the evaluation methods.

In current clinical medicine, among cardiomyocytes immediately after differentiation, those that are expected to grow as cardiomyocytes are formed into a sheet shape, and then the cells in the sheet are matured. For evaluation of the degree of differentiation of cardiomyocytes immediately after differentiation, cell fixation treatment is performed, after which the cells are stained with a TnT2 antibody which is a cardiomyocyte marker, and the degree of differentiation is evaluated by fluorescence activated cell sorting (FACS).

After the evaluation, the quality of the cardiomyocytes is analyzed by the level of pulsation of the cardiomyocyte sheet which is associated with maturation of the cardiomyocytes, and the drug response of the cardiomyocytes. For analysis of the quality, microscope observation of fluorescently stained cells and FACS, and quantitative polymerase chain reaction (PCR) on nucleic acids extracted from the cells are applied. These analysis methods are all invasive to cardiomyocytes. Alternatives to these analysis methods are also invasive to cardiomyocytes.

When cardiomyocytes immediately after differentiation and sheet-shaped cardiomyocytes are used in clinical medicine, cardiomyocytes which have undergone evaluation by any of the above-described invasive methods are difficult to apply on their own to a living organism. Therefore, there is no option other than tests using a sample independent of cells to be applied to a living organism. For this reason, a noninvasive method for evaluation of cardiomyocytes immediately after differentiation and cardiomyocytes artificially formed into a sheet shape is required. The same applies to a case where cardiomyocytes having a shape other than a sheet shape are used in clinical medicine.

Examples of the related art related to evaluation of cardiomyocytes by Raman spectroscopy include the followings.

Patent Literature 1, paragraph [0038], indicates that a Raman spectrum of the cytoplasm of cardiomyocytes obtained from the subepicardial myocardium of a rat was measured. The Raman spectrum includes Raman bands at 751 cm⁻¹, 1,130 cm⁻¹ and 1,582 cm⁻¹. These Raman bands may be vibrational modes derived from a porphyrin ring present at the center of heme. The peaks for reduced cytochrome b5 and reduced cytochrome c, among heme proteins concerned, closely match the peaks for the cardiomyocytes.

In Non Patent Literature 1, C2C12 mouse striated myocytes whose shape is significantly changed due to differentiation are evaluated using Raman scattering. Here, a Raman scattering image of the cells is acquired using a peak of cytochrome c at 753 cm⁻¹.

In Non Patent Literature 2, a Raman shift at 570 cm⁻¹ is observed in a Raman spectrum of myoglobin extracted from a horse heart.

### Citation List

### Patent Literature

Patent Literature 1: International Patent Publication No. 2010/103661
Patent Literature 2: Japanese Unexamined Patent Application Publication No. 2020/018242
Patent Literature 3: International Patent Publication No. 2020/067479
Patent Literature 4: International Patent Publication No. 2013/111875
Patent Literature 5: International Patent Publication No. 2016/060260
Patent Literature 6: International Patent Publication No. 2018/124210

### Non Patent Literature

Non Patent Literature 1: Ichimura, T., Chiu, Ld., Fujita, K. et al. Visualizing the appearance and disappearance of the attractor of differentiation using Raman spectral imaging. Sci Rep 5, 11358 (2015). [online], [retrieved on 2021-10-19]. Retrieved from <https://doi.org/10.1038/srep11358>
Non Patent Literature 2: Jeyarajah S, Proniewicz LM, Bronder H, Kincaid JR., "Low frequency vibrational modes of oxygenated myoglobin, hemoglobins, and modified derivatives", J Biol Chem, 1994 Dec 9;269(49):31047-50, [online], [retrieved on 2021-06-03]. Retrieved from <https://doi.org/10.1016/S0021-9258(18)47388-7>
Non Patent Literature 3: Giacomelli, Elisa et al., "Human-iPSC-Derived Cardiac Stromal Cells Enhance Maturation in 3D Cardiac Microtissues and Reveal Non-cardiomyocyte Contributions to Heart Disease", May 26, 2020, Cell Stem Cell, Volume 26, Issue 6, 862-879, e11, [online], [retrieved on 2021-06-03]. Retrieved from <https://doi.org/10.1016/j.stem.2020.05.004>
Non Patent Literature 4: Anton Mihic, Jiao Li, Yasuo Miyagi, Mark Gagliardi, Shu-Hong Li, Jean Zu, Richard D. Weisel, Gordon Keller, Ren-Ke Li, "The effect of cyclic stretch on maturation and 3D tissue formation of human embryonic stem cell-derived cardiomyocytes", Biomaterials, Volume 35, Issue 9, 2014, Pages 2798-2808, [online], [retrieved on 2021-06-03]. Retrieved from <https://doi.org/10.1016/j.biomaterials.2013.12.052>
Non Patent Literature 5: Nunes, S., Miklas, J., Liu, J. et al. "Biowire: a platform for maturation of human pluripotent stem cell-derived cardiomyocytes", Nat Methods 10, 781-787 (2013), [online], [retrieved on 2021-06-03]. Retrieved from <https://doi.org/10.1038/nmeth.2524>
Non Patent Literature 6: Amandine F.G. Godier-Furnemont, Malte Tiburcy, Eva Wagner, Matthias Dewenter, Simon Lammle, Ali El-Armouche, Stephan E. Lehnart, Gordana Vunjak-Novakovic, Wolfram-Hubertus Zimmermann, "Physiologic force-frequency response in engineered heart muscle by electromechanical stimulation", Biomaterials, Volume 60, 2015, Pages 82-91, [online], [retrieved on 2021-06-03]. Retrieved from <https://doi.org/10.1016/j.biomaterials.2015.03.055>
Non Patent Literature 7: Xi Lou, Meng Zhao, Chengming Fan, Vladimir G Fast, Mani T Valarmathi, Wuqiang Zhu, Jianyi Zhang, "N-cadherin overexpression enhances the reparative potency of human-induced pluripotent stem cell-derived cardiac myocytes in infarcted mouse hearts", Cardiovascular Research, Volume 116, Issue 3, 1 March 2020, Pages 671-685, [online], [retrieved on 2021-11-05]. Retrieved from <https://doi.org/10.1093/cvr/cvz179>
Non Patent Literature 8: Feyen, Dries A.M. et al., "Metabolic Maturation Media Improve Physiological Function of Human iPSC-Derived Cardiomyocytes", Cell Reports, Volume 32, Issue 3, 107925, [online], [retrieved on 2021-06-03]. Retrieved from <https://doi.org/10.1016/j.celrep.2020.107925>
Non Patent Literature 9: Antje Ebert, Amit U. Joshi, Sandra Andorf, Yuanyuan Dai, Shrivatsan Sampathkumar, Haodong Chen, Yingxin Li, Priyanka Garg, Karl Toischer, Gerd Hasenfuss, Daria Mochly-Rosen, Joseph C. Wu, "Proteasome-Dependent Regulation of Distinct Metabolic States During Long-Term Culture of Human iPSC-Derived Cardiomyocytes", Circulation Research. 2019;125:90-103, 20 May 2019, [online], [retrieved on 2021-06-03]. Retrieved from <https://doi.org/10.1161/CIRCRESAHA.118.313973>

### Summary of Invention

### Technical Problem

The present invention provides methods for evaluating differentiation into cardiomyocytes and maturation of cardiomyocytes.

### Solution to Problem

<1> A method for evaluating cardiomyocytes using Raman scattering, the method including:
   acquiring a Raman spectrum of cardiomyocytes artificially induced to differentiate from pluripotent stem cells;
   acquiring an intensity of Raman-scattered light for a protein containing at least one of heme b and heme c as a prosthetic group from the Raman spectrum; and
   evaluating a state of progress of maturation of the cardiomyocytes on the basis of the intensity of the Raman-scattered light.
<2> The method according to <1>, in which
   the protein containing at least one of heme b and heme c as a prosthetic group is reduced cytochrome c, and
   the intensity of the Raman-scattered light, which is Raman-scattered light for the reduced cytochrome c is acquired, and a peak of the intensity is calculated on the basis of an intensity of Raman-scattered light at a Raman shift of 743 cm⁻¹ to 755 cm⁻¹ in wavenumber.
<3> The method according to <1>, in which
   the protein containing at least one of heme b and heme c as a prosthetic group is oxymyoglobin, and
   an intensity of the Raman-scattered light for the oxymyoglobin is acquired, and the intensity is calculated on the basis of at least an intensity of Raman-scattered light at a Raman shift of 565 cm⁻¹ to 575 cm⁻¹ in wavenumber.
<4> The method according to any one of <1> to <3>, in which the Raman spectrum excited by light having a wavelength of 532 nm is used.
<5> The method according to any one of <1> to <4>, in which
   an intensity of Raman-scattered light for lipid is further acquired from the Raman spectrum, and
   a state of progress of maturation of cardiomyocytes is evaluated on the basis of the intensity of the Raman-scattered light for lipid.
<6> The method according to any one of <1> to <5>, in which the pluripotent stem cells are human cells.
<7> The method according to <6>, in which the cardiomyocytes aggregate into a sheet-shaped tissue fragment.
<8> The method according to <6>, in which the cardiomyocytes aggregate around a core to form a cell mass.
<9> The method according to <6>, in which
   the cardiomyocytes form a spheroid with human cardiac fibroblast cells, human cardiac endothelial cells and human mesenchymal cells, or
   the cardiomyocytes form a spheroid with human cardiac fibroblast cells and human cardiac endothelial cells.
<10> The method according to <6>, in which the cardiomyocytes form a cardiac organoid.
<11> The method according to <6>, further including, before acquiring the Raman spectrum, accelerating maturation of the cardiomyocytes by at least one of:
   application of a mechanical stimulus to the cardiomyocytes;
   application of an electrical stimulus to the cardiomyocytes;
   introduction of a gene to the cardiomyocytes;
   co-culture of the cardiomyocytes with other cells; and
   addition of an oxidizing substrate, a compound and a factor to a medium in which the cardiomyocytes are cultured.
<12> The method according to any one of <6> to <11>, in which acquiring the Raman spectrum, acquiring the intensity of the Raman-scattered light and evaluating the state of progress of maturation is repeatedly applied to the same cardiomyocytes over time.
<13> The method according to any one of <6> to <12>, further including, before acquiring the Raman spectrum, culturing the cardiomyocytes in the presence of a drug, in which
   the state of progress of maturation of the cardiomyocytes is evaluated to evaluate an effect of the drug on maturation of the cardiomyocytes.
<14> The method according to any one of <1> to <13>, in which the pluripotent stem cells are one of embryonic stem cells (ESCs), induced pluripotent stem cells (iPSCs) and embryonic germ cells (EGCs), and
   the embryonic stem cells (ESCs) include nuclear transfer embryonic stem cells (ntESCs).
<15> The method according to any one of <1> to <14>, in which a peak of excitation light in the acquisition of the Raman spectrum is in a range of 400 nm to 600 nm.
<16> The method according to any one of <1> to <15>, in which a temperature of the cardiomyocytes is decreased to 4°C or lower in the acquisition of the Raman spectrum.
<17> A method for evaluating differentiation into cardiomyocytes using Raman scattering, the method including:
   artificially inducing cells, which are pluripotent stem cells, to differentiate into cardiomyocytes;
   acquiring a Raman spectrum of the cells induced to differentiate;
   acquiring an intensity of Raman-scattered light for at least one of heme b and heme c from the Raman spectrum; and
   evaluating a state of progress of differentiation of the cells into cardiomyocytes on the basis of the intensity of the Raman-scattered light.

### Advantageous Effects of Invention

The present invention provides methods for evaluating differentiation into cardiomyocytes and maturation of cardiomyocytes.

### Brief Description of Drawings

Fig. 1 shows a flow of an evaluation method.
Fig. 2 is a schematic diagram of Raman spectroscopy on a cardiomyocyte sheet.
Fig. 3 shows Raman spectra.
Fig. 4 shows distributions of intensity of Raman-scattered light for a cardiomyocyte sheet.
Fig. 5 shows a temporal shift in intensity of Raman-scattered light at a Raman shift of 570 cm⁻¹ in wavenumber.
Fig. 6 shows a temporal shift in intensity of Raman-scattered light at a Raman shift of 748 cm⁻¹ in wavenumber.
Fig. 7 shows gene expression levels of myoglobin.
Fig. 8 shows gene expression levels of cytochrome c.
Fig. 9 shows images of fluorescently stained cardiomyocyte sheets.
Fig. 10 shows evaluation of the differentiation degree by Raman spectroscopy.
Fig. 11 is a schematic diagram of Raman spectroscopy on a cell mass.
Fig. 12 is a schematic diagram of Raman spectroscopy on a well plate.
Fig. 13 is a schematic diagram of Raman spectroscopy in microchannels.

### Description of Embodiments

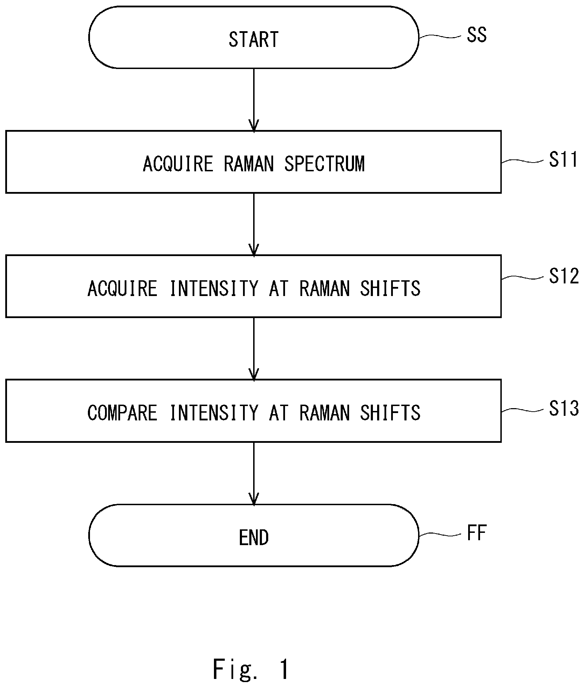

Fig. 1 shows a flow of a method for evaluating cardiomyocytes using Raman scattering according to an aspect of the present invention. The evaluation items are states of progress of differentiation and maturation of cardiomyocytes. In step S11, a Raman spectrum of cardiomyocytes is acquired. Details of the Raman spectrum will be described below.

In an aspect of the present invention, the cardiomyocytes, for which the Raman spectrum is to be acquired, are cardiomyocytes in the process of being artificially induced to differentiate from pluripotent stem cells, or cardiomyocytes having already been induced to differentiate. In an aspect of the invention, the cardiomyocytes are cardiomyocytes in the process of being induced to differentiate from pluripotent stem cells with the pluripotent stem cells being artificially cultured, or cardiomyocytes having already been induced to differentiate. In an aspect of the invention, the pluripotent stem cells are human cells.

The pluripotent stem cells are cells which can be differentiated into almost all cells that form a body. The pluripotent stem cells are classified into several types. As pluripotent stem cells that have been established to date, embryonic stem cells (ESCs), embryonic germ cells (EGCs) and induced pluripotent stem cells (iPSCs) are known. Examples of the pluripotent stem cells to which the evaluation method of the present invention is applied include, but are not limited to, embryonic stem cells (ESCs), embryonic germ cells (EGCs) and induced pluripotent stem cells (iPSCs). The embryonic stem cells (ESCs) include nuclear transfer embryonic stem cells (ntESCs) in addition to common embryonic stem cells.

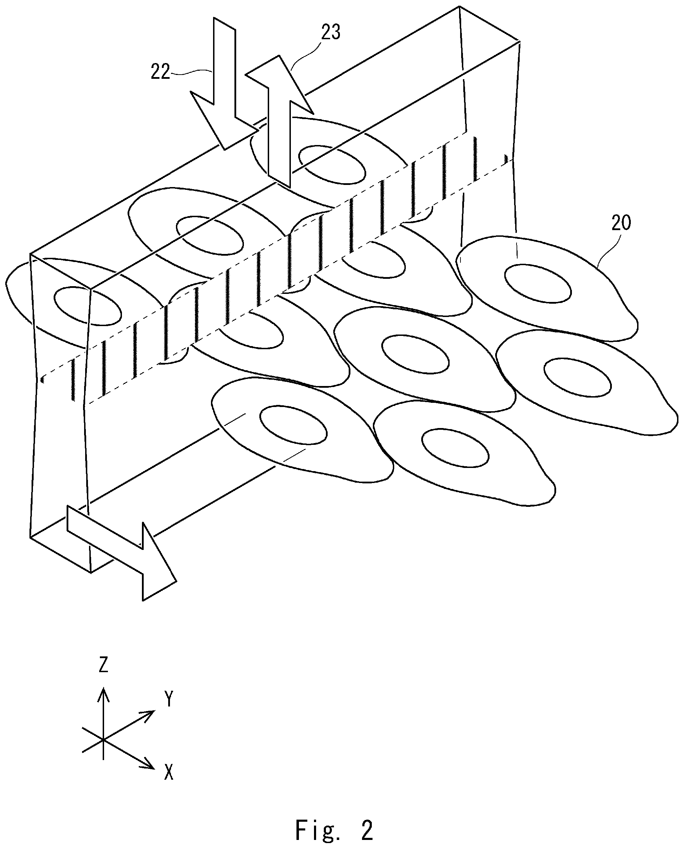

Fig. 2 shows a schematic diagram of Raman spectroscopy on a cell sheet 20. In the present aspect, a cardiomyocyte sheet is taken as an example, and methods for evaluation thereof are described, but the form of cardiomyocytes is not limited thereto. In an aspect different from that shown in Fig. 2, the cardiomyocytes are in any of the forms of a cell sheet, a cell mass, and cells that do not bind to one another, but are dispersed in isolation. The cell sheet 20 may be replaced by, for example, a group of cells before formation of the sheet, which is disposed on a substrate.

In Fig. 2, excitation light 22 is focused on a line parallel to the y axis through an objective lens (not shown). The focused excitation light 22 is scanned on the cell sheet 20. In the figure, the scanning is performed in the +x direction. The scanning causes Raman scattering in a relatively wide region of, for example, 100 µm square on the cell sheet.

In an aspect of the present invention which is shown in Fig. 2, the excitation light 22 has a peak preferably at a wavelength of 400 nm to 600 nm, more preferably at a wavelength of 406 nm to 561 nm, particularly preferably at 532 nm. In an aspect thereof, the excitation light 22 is laser light. The wavelength of the excitation light 22 is also suitable for excitation light that is used in various other Raman spectrometric methods described later.

In the aspect of the present invention which is shown in Fig. 2, the excitation light 22 gives intense heat to the cell sheet 20. The cardiomyocytes in the cell sheet 20 may be damaged by heat from the excitation light 22. In scanning of the excitation light 22 on the cell sheet 20, the cell sheet 20 is cooled to decrease the temperature of the cell sheet 20 to a temperature lower than a temperature at which the cell sheet 20 is cultured, for example, 37°C. For example, the temperature of the cell sheet 20 is decreased to 4°C or lower. This suppresses damage to the cardiomyocytes. In addition, the decreasing of the temperature of the cell sheet 20 reduces the frequency of pulsation of the cardiomyocytes. This suppresses occurrence of displacement of the sample in scanning of the excitation light 22 on the cell sheet 20. By decreasing the temperature to a temperature lower than 4°C, the peaks of a Raman spectrum acquired from the cell sheet 20 can be further sharpened. This improves the wavenumber resolution, so that it is easy to perform spectral separation. In an aspect, the temperature of the cell sheet 20 is decreased to a minimum temperature at which the cells are not frozen.

In Fig. 2, a spectrum of Raman-scattered light 23 generated in the cell sheet 20 is detected and recorded. The spectrum is detected by making the Raman-scattered light pass through a spectroscope or a band pass filter, followed by detection of the light with a two-dimensional light detector. In this way, a two-dimensional image of the Raman spectrum on the cell sheet 20 is acquired. Thus, the Raman spectrum is observed with a microscope.

The cytoplasm of cells just after completion of differentiation into cardiomyocytes has a composition inherent in cardiomyocytes unlike the cytoplasm of cells before differentiation. Cells just after completion of differentiation into cardiomyocytes are different in gene and protein expression levels from cells before differentiation. Cells just after completion of differentiation into cardiomyocytes have a myocardial function. Therefore, cells just after completion of differentiation into cardiomyocytes present a Raman shift different from that of cells before differentiation. It is useful to observe a Raman spectrum with a microscope on cells which are being induced to differentiate from pluripotent stem cells.

On the other hand, cells just after completion of differentiation into cardiomyocytes are immature as cardiomyocytes. Immature cardiomyocytes and matured cardiomyocytes are different in gene expression level, protein expression level and myocardial function level. Cells matured in an accelerative manner before the acquisition of a Raman spectrum present a Raman shift different from that of immature cells.

Evaluated by the present evaluation method is the degree of differentiation of cells before differentiation into cardiomyocytes, which is or may be induced by a physical method, a biological method and a chemical method. The cells may be induced to differentiate in accordance with a method for producing cardiomyocytes as described in Patent Literature 2 and a method for producing a multi-layered myocardial tissue culture as described in Patent Literature 3. That is, first, an embryoid is cultured in the form of a spheroid. Next, the embryoid is induced to differentiate into cardiomyocytes while being three-dimensionally suspension-cultured.

Evaluated by the present evaluation method is the degree of maturation of cardiomyocytes, which is or may be accelerated by a physical method, a biological method and a chemical method. The cardiomyocytes are cells that have already been induced to differentiate.

The physical method includes, but is not limited to, mechanical stimulation, and electrical stimulation of immature cardiomyocytes. The biological method includes, but is not limited to, introduction of a gene to immature cardiomyocytes, and co-culture with cells other than cardiomyocytes. The chemical method includes, but is not limited to, addition of an oxidizing substrate and other compounds to a medium in which immature cardiomyocytes are cultured. In other aspects, the chemical method includes addition of an oxidizing substrate and other factors to a medium in which immature cardiomyocytes are cultured.

In other aspects of the chemical method, pluripotent stem cells are cultured in a medium containing a WNT signal activator, and then in a medium containing a WNT signal inhibitor. Examples of the WNT signal activator include GSK3β inhibitors such as BIO and CHIR99021. Examples of the WNT signal inhibitor include those consisting of a low-molecular compound or a protein, for example, KY02111, IWP-2, IWR-1, XAV939, DKK1 and IGFBP.

Proteins having heme as a prosthetic group include cytochrome, and a protein called myoglobin.

Cytochrome containing heme b is called cytochrome b, and cytochrome containing heme c is called cytochrome c. Cytochrome b and cytochrome c each includes an oxidized form and a reduced form.

### <Structural formula of heme b>

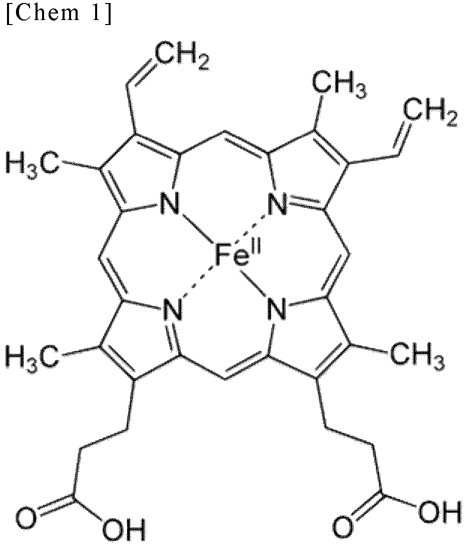

### <Structural formula of heme c>

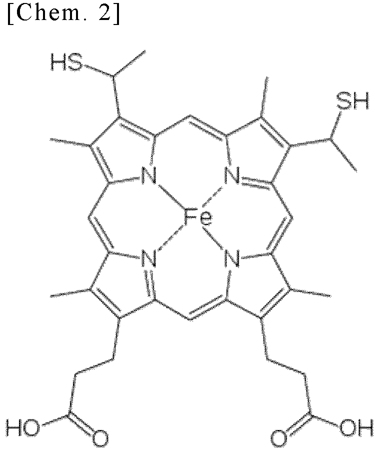

The myoglobin is a heme protein having heme b as a prosthetic group. The myoglobin is further classified into reduced myoglobin, oxymyoglobin (oxygen-type myoglobin), and other myoglobins.

Refer back to Fig. 1. In step S12, an intensity of Raman-scattered light at a Raman shift, that is, a predetermined wavenumber of Raman shift is acquired from the Raman spectrum. Here, the predetermined wavenumber of Raman shift is specific to at least one of reduced cytochrome c and oxymyoglobin (oxygen-type myoglobin).

In an aspect of the present invention, an intensity of Raman-scattered light for at least reduced cytochrome c is acquired. The intensity of Raman-scattered light is calculated on the basis of an intensity of Raman-scattered light at a Raman shift of 743 to 755 cm⁻¹ in wavenumber, preferably at a Raman shift of 748 cm⁻¹ in wavenumber.

In an aspect of the present invention, an intensity of Raman-scattered light for at least oxymyoglobin is acquired. The intensity of Raman-scattered light is calculated on the basis of an intensity of Raman-scattered light at a Raman shift of 565 to 575 cm⁻¹ in wavenumber, preferably at a Raman shift of 570 cm⁻¹ in wavenumber.

By Raman spectroscopy, the intensities of Raman-scattered light for a plurality of types of molecules including the reduced cytochrome c and oxymyoglobin are acquired on the same cardiomyocytes in one measurement. As an example, the intensity of Raman-scattered light for lipid is acquired. This helps to perform analysis across a plurality of events in the same cells. On the other hand, conventional fluorescent analysis performed on cardiomyocytes, and diagnosis by pulsation do not enable evaluation of correlations between a plurality of biological molecules in the same cardiomyocytes. Analysis performed across a plurality of events in the same cells by Raman spectroscopy helps to analyze the effect of the drug, the toxicity of the drug and the metabolism of the drug.

In step S13 shown in Fig. 1, the intensity of Raman-scattered light acquired in step S12 is compared to data. Here, the data includes a correlation between the intensity of Raman-scattered light and the state of progress of maturation of cardiomyocytes. The term "maturation" as used herein refers to the process from immature cardiomyocytes to matured cardiomyocytes. Data may be acquired previously, or may be acquired cardiomyocytes as a positive control and a negative control in parallel to Raman spectroscopy on cardiomyocytes of interest. The acquired Raman intensity and the data are compared to evaluate the state of progress of maturation of the cardiomyocytes to be tested.

As described above, cardiomyocytes confirmed to be matured are transplanted into a living organism, or applied to drug discovery evaluation. The drug discovery evaluation includes, but is not limited to, analysis of the effect of the drug, the toxicity of the drug and the metabolism of the drug. Here, before application of the cardiomyocytes to the living organism, the temperature of the cardiomyocytes is maintained lower than a temperature at which the cardiomyocytes are cultured. Application of cardiomyocytes to a living organism includes, but is not limited to, transplantation of cardiomyocytes into a living organism. Preferably, the cardiomyocytes are maintained at a temperature of 4°C. This inhibits the cardiomyocytes from being damaged before the transplantation of the cardiomyocytes into the living organism even after evaluation of the state of progress of maturation of the cardiomyocytes.

In other aspects, in step S13 shown in Fig. 1, the intensity of Raman-scattered light which is acquired in step S12 is compared to the data. Here, the data includes a correlation between the intensity of Raman-scattered light and the state of progress of differentiation of the cells. The term "differentiation" as used herein refers to the process from undifferentiated cells, for example, pluripotent stem cells to immature cardiomyocytes. The data may be acquired previously, or may be acquired from cells as a positive control and a negative control in parallel to Raman spectroscopy on cells of interest. The acquired Raman intensity and the data are compared to evaluate the state of progress of differentiation of the cardiomyocytes to be tested. It is also possible to obtain matured cardiomyocytes by further inducing cardiomyocytes confirmed to be differentiated as described above.

### Experiment Examples

Hereinafter, the aspects of the present invention will be described with reference to actually implemented Experiment Examples. The following description is not intended to limit the scope of the present invention, and is merely a sample for promoting a better understanding of the technical idea of the present invention.

A cardiomyocyte sheet was prepared according to Patent Literature 4.
That is, first, on a culture dish, a human iPS cell line 253G1 (Cell No. HPS0002, RIKEN, Cell Engineering Division -CELL BANK-, Japan) was induced to differentiate into cardiomyocytes (CMs). For differentiation and induction, first, pluripotent stem cells were cultured in a medium containing a WNT signal activator. Next, the cells were cultured in a medium containing a WNT signal inhibitor. One week after the differentiation and induction, pulsation was confirmed in the cells. Two weeks after the differentiation and induction, cells were collected.

A part of the collected cells is stained with a TnT2 fluorescent antibody which is a cardiomyocyte-specific marker (SC-20025; Santa Cruz Biotechnology, Dallas, TX, USA). After the staining, the efficiency of the differentiation and induction was examined by FACS. As a control, cells in process of differentiation were collected, and similarly analyzed by FACS.

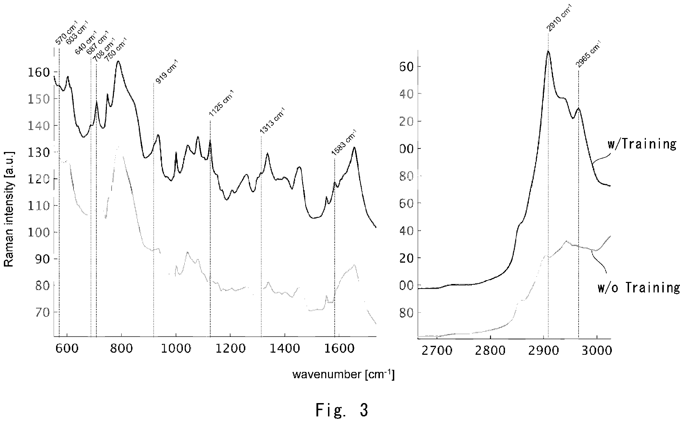

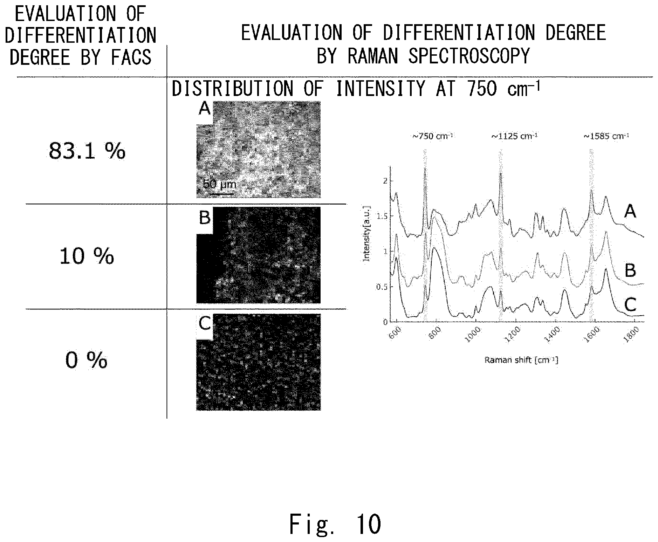

Further, training was applied to cells 2 weeks after the differentiation and induction. The cells were stained, followed by examination of the state of maturation by FACS and Raman spectroscopy. As a control, cells to which training had not been applied, were similarly analyzed by FACS and Raman spectroscopy. The results of observation concerning maturation of the cardiomyocytes are shown in Figs. 3 to 9. The results of observation on differentiation into cardiomyocytes are shown in Fig. 10.

Training was performed on a part of the prepared cardiomyocyte sheets. In the training, maturation of cardiomyocytes was physically accelerated by mechanical stimulation or electrical stimulation.

While the training was performed, cardiomyocyte sheets on which training was not performed were not treated, and were stored while being continuously cultured in an incubator at a temperature of 37°C.

Fig. 3 shows Raman spectra acquired within a predetermined region on the cell sheet. The horizontal axis represents a Raman shift in wavenumber [cm⁻¹]. The vertical axis represents an intensity of Raman-scattered light, that is, a Raman intensity [a.u.]. The two curves drawn represent, respectively, a Raman spectrum of a cardiomyocyte sheet which underwent maturation through 2-week training (w/ training) and a Raman spectrum of a cardiomyocyte sheet which did not undergo maturation through training (w/o training).

The Raman spectra were acquired in the manner described with reference to Fig. 2. Raman peaks appear in the Raman spectrum. The Raman peak represents an increase in intensity of Raman-scattered light at a corresponding Raman shift. At Raman shifts represented by the following wavenumbers, the corresponding Raman intensity increases after maturation through training.

A Raman shift derived from oxymyoglobin is observed at a wavenumber of about 570 cm⁻¹. See descriptions in Non Patent Literature 2.

Peaks observed at wavenumbers of about 595 to 605 cm⁻¹, about 635 to 645 cm⁻¹, about 685 to 695 cm⁻¹, about 743 to 755 cm⁻¹, about 915 to 925 cm⁻¹, about 1120-1130 cm⁻¹, about 1310 to 1320 cm⁻¹ and about 1580 to 1590 cm⁻¹ indicate an increase in Raman signals due to maturation through training. These peaks may correspond to Raman shifts derived from oxidized and reduced cytochrome b and oxidized and reduced cytochrome c.

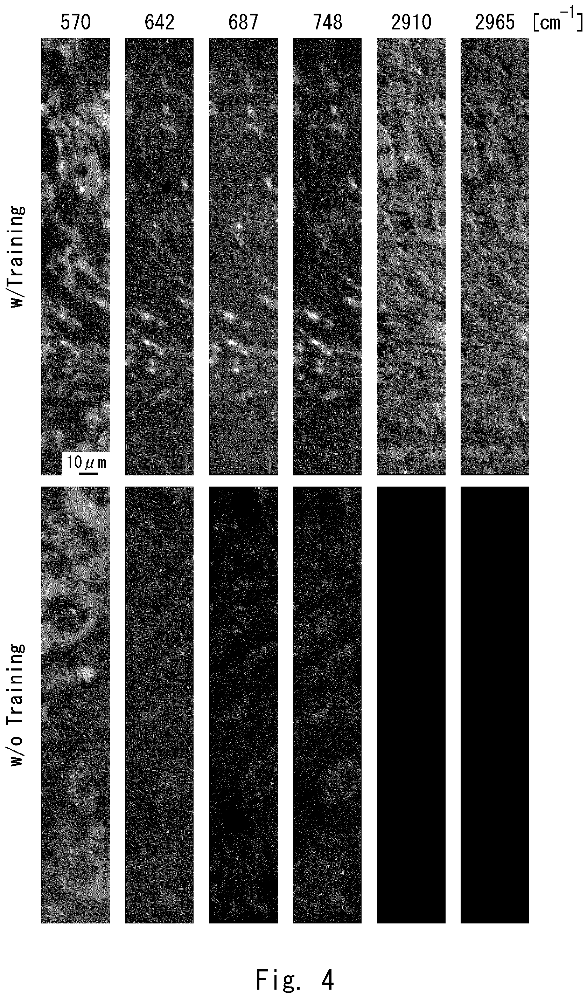

Fig. 4 shows distributions of intensity of Raman-scattered light at a specific wavenumber of Raman shift on a cell sheet, which is a so-called Raman image. A cardiomyocyte sheet which underwent maturation through training (w/ training) is shown on the upper side. A cardiomyocyte sheet which did not undergo maturation through training (control, i.e., w/o training) is shown on the lower side.

The columns show
Raman images at a Raman shift of about 570 cm⁻¹ in wavenumber,
Raman images at a Raman shift of about 642 cm⁻¹ (640 cm⁻¹) in wavenumber,
Raman images at a Raman shift of about 687 cm⁻¹ in wavenumber,
Raman images at a Raman shift of about 748 cm⁻¹ in wavenumber,
Raman images at a Raman shift of about 2910 cm⁻¹ in wavenumber, and
Raman images at a Raman shift of about 2965 cm⁻¹ in wavenumber,
in the stated order from the left.

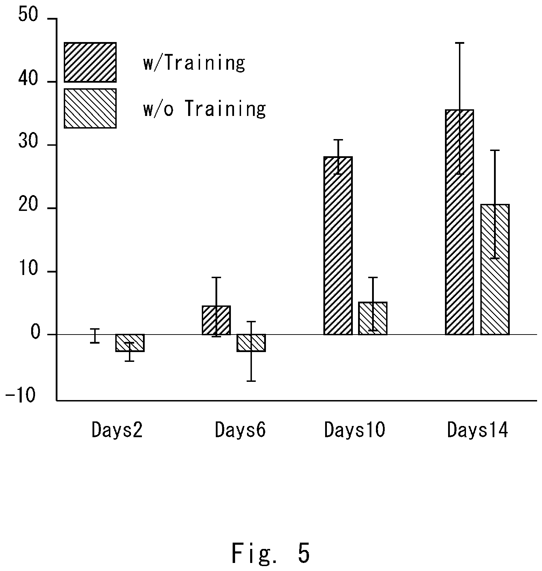

Fig. 5 shows a temporal shift in Raman shift signal intensity, that is, Raman intensity, at a wavenumber of about 570 cm⁻¹. The cardiomyocyte sheets used in observation were distributed to four culture dishes. On the culture dishes, culture and training were started (w/ training). The culture dishes were each observed 2 days after the start of training (Day 2), 6 days after the start of training (Day 6), 10 days after the start of training (Day 10), and 14 days after the start of training (Day 14). Culture cells as a control (w/o training) were similarly distributed, and similarly observed after being cultured for the same periods.

The Raman intensity was calculated from the Raman image in the following manner. First, four arithmetic operations were performed on the Raman shift signal intensity at the above-described wavenumber. The four arithmetic operations were performed as follows: the area of a region surrounded by straight lines connecting the apex of an arbitrary Raman peak and two bottom points sandwiching the peak in the Raman spectrum is calculated, and taken as a signal intensity. The calculation means addition of peak signals and subtraction of background signals. Before and after training, the area is calculated for Raman peaks at other wavenumbers, which have a positive correlation with the signal intensity, and further, addition of, for example, peak signals which are derived from the same molecule but are different in wavenumber because of different vibrational modes may be performed to accentuate peak signals. Cells that have undergone training and cells that have not undergone training may be compared on the basis of the number of cells and the density of cells. Here, for performing the comparison by quantitative evaluation, a Raman signal representing a molecular vibration which does not change the signal amount as Raman intensity before and after training, for example, a Raman signal of the vibration of phenylalanine is set to a basis for comparison. Here, multiplication or division is performed to normalize the intensity of the Raman signal. The calculation is performed on coordinates on the measured cardiomyocyte sheet to obtain a Raman image.

Next, in a spatial coordinate system obtained by adding the intensity of the Raman signal to the plane of the Raman image, an average of Raman signals is obtained for the Raman image to calculate the Raman intensity in a predetermined range on the cardiomyocyte sheet. The operation for obtaining the average in the spatial coordinate system is carried out in the following manner. First, a coordinate having Raman signals is extracted on the plane of the Raman image acquired as described above. Here, the Raman image includes a Raman peak signal intensity distribution. The extraction is performed with an arbitrary signal intensity set as a threshold on the Raman peak. By the extraction, an average intensity per pixel is calculated. As another method, an average Raman intensity for all coordinates within a predetermined region on the plane of the acquired Raman image is calculated. An average Raman intensity for all coordinates on the plane of the acquired Raman image may be calculated.

The Raman intensity at a Raman shift of about 570 cm⁻¹ in wavenumber, which indicates the presence of oxymyoglobin, increased with time in both w/ training and the control, i.e., w/o training. The intensity was further increased by maturation through training.

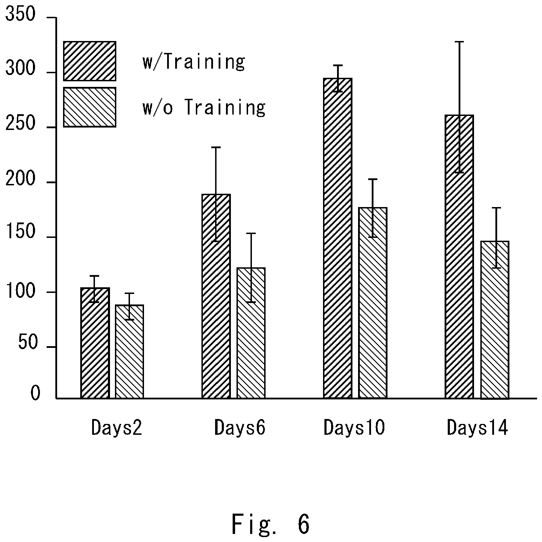

Fig. 6 shows a temporal shift in intensity of Raman-scattered light at 748 cm⁻¹. The Raman intensity at a Raman shift of about 748 cm⁻¹ in wavenumber, which indicates the presence of reduced hemes c and b, in particular, reduced cytochrome c and cytochrome b, increased with time in both w/ training and the control, i.e., w/o training, and peaked around day 10. The intensity was further increased by maturation through training.

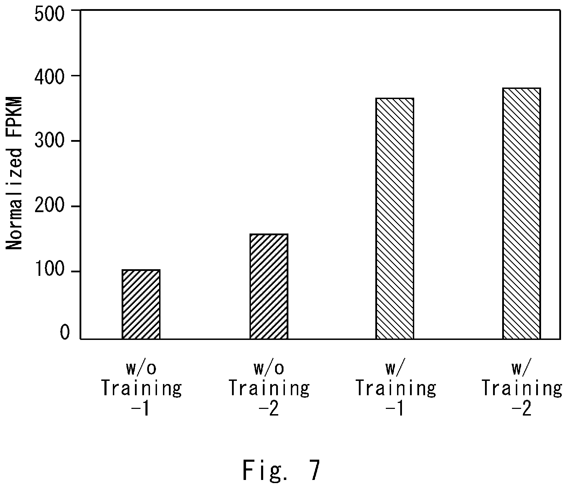

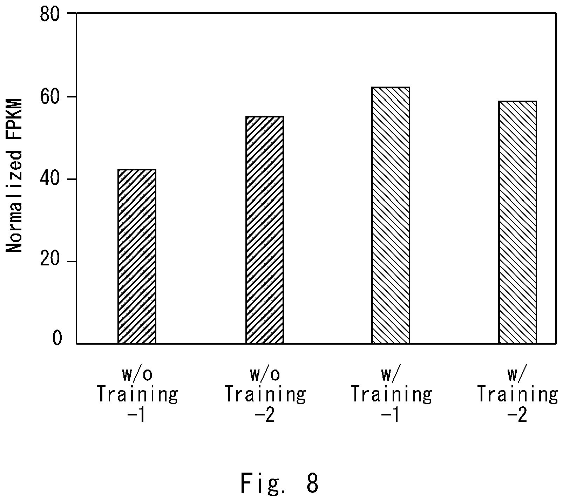

Fig. 7 shows gene expression levels of myoglobin in the cardiomyocyte sheet. Fig. 8 shows gene expression levels of cytochrome c. The w/o training-1 and the w/o training-2 as controls each represent an independent trial using a cardiomyocyte sheet that did not undergo maturation through training. The w/ training-1 and the w/ training-2 each represent an independent trial using a cardiomyocyte sheet that underwent maturation through training.

The gene expression level was measured by a total gene analysis method. That is, total RNA was extracted from the cardiomyocyte sheet using a Trizol reagent (Life Technologies Corporation). RNA-Seq data was acquired with Illumina Hiseq 4000 Platform (Illumina, Inc.) to perform sequence analysis of cDNA. Here, the vertical axis "Normalized FRKM" of the graph shown in each of Figs. 7 and 8 has the following meaning. First, referring to read count data obtained from RNA-Seq, it can be seen that the amount of a transcription product represents a gene expression level itself. Data obtained by performing normalization in terms of the length of a transcription product after read count data obtained from RNA-Seq data is corrected with the total number of reads is referred to as FPKM (PRKM in the case of a single-end read). FPKM is an abbreviation of "fragments per kilobase of exon per million reads mapped".

In w/ training-1 and w/ training-2, the gene expression of cytochrome c and myoglobin in cardiomyocytes increased.

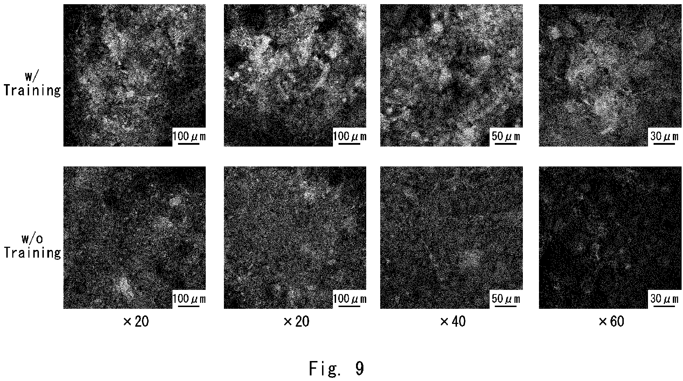

Fig. 9 shows images of fluorescently stained cardiomyocyte sheets. The w/ training represents a cardiomyocyte sheet that underwent maturation through training. The w/o training represents a cardiomyocyte sheet that did not undergo maturation through training.

The cardiomyocytes were fluorescently stained and images thereof were acquired in the following manner. The cardiomyocytes were treated with 4% paraformaldehyde at room temperature for 30 minutes to be fixed. To D-PBS, 0.5% (v/v) Triton X-100 was added previously, and membrane permeation treatment was performed for 1 hour. Next, the cardiomyocytes were treated at 4°C for 16 hours using a blocking solution (0.1% (v/v) Tween-20, 5% (v/v) normal donkey serum, 3% (v/v) bovine serum albumin, 5% (v/v) normal goat serum, D-PBS). An anti-troponin antibody as a primary antibody and cardiomyocytes were reacted. Thereafter, the cells were washed with PBS, and reacted at room temperature for 1 hour with a secondary antibody diluted to 1 : 300 with a blocking buffer. Finally, the cells were reacted with 4'-6-diamidino-2-phenylindole (DAPI; 300 nM; Wako Pure Chemical Industries, Ltd.) at room temperature for 30 minutes to stain cell nuclei.

Light sections of the images shown in Fig. 9 indicate localized cytochrome c. In w/ training, the amount of cytochrome c in the cardiomyocytes increased.

An increase in gene expression cytochrome c and myoglobin and abundance of cytochrome c molecules, which is shown in Figs. 7 to 9, is consistent with an increase in intensity of Raman-scattered light.

From Experiment Example above, it can be seen that by using Raman spectroscopy, the degree of maturation of cardiomyocytes can be noninvasively evaluated and diagnosed. In the Raman spectroscopy according to Experiment Example above, Raman scattering that reflects molecular vibration of molecules themselves in cardiomyocytes was detected. There, the compositions of molecules in cardiomyocytes before maturation were analyzed in a manner noninvasive to the cardiomyocytes.

As described in Background Art, it has not been heretofore possible to noninvasively diagnose the degree of maturation of cardiomyocytes. On the other hand, methods using Raman spectroscopy are noninvasive. Thus, a process in which cardiomyocytes are matured from the early stage of maturation can be observed over time for the same cardiomyocytes. Therefore, methods using Raman spectroscopy are useful for studies on maturation of cardiomyocytes. In methods using Raman spectroscopy, the quality of cardiomyocytes to be transplanted can be evaluated without labeling the cardiomyocytes.

Next, an example in which Raman spectroscopy was applied to measurement of differentiation into cardiomyocytes will be shown. Fig. 10 shows an example of evaluation of the differentiation degree by Raman spectroscopy. iPS cells were differentiated into cardiomyocytes. Three types of cells different in differentiation degree were evaluated by Raman spectroscopy. The results of evaluating the differentiation degree by evaluating the cell groups by FACS are shown in the left column. A cell group is determined to have been differentiated into cardiomyocytes from a state before differentiation when the differentiation degree is evaluated as being more than 80% in FACS. Further, the intensity of Raman-scattered light for each cell group was measured. Average spectra in regions measured are shown in the right column.

There was a positive correlation between differentiation degrees determined from the results of the FACS measurement and the magnitudes of specific Raman peaks derived from cytochrome c as shown in Fig. 10. The Raman shifts for these Raman peaks were about 750, 1125 and 1585 cm⁻¹. The above results show that the degree of differentiation into cardiomyocytes from cells before differentiation can be noninvasively measured on the basis of an increase in Raman signals.

From Experiment Example above, it can be seen that by using Raman spectroscopy, the degree of differentiation into cardiomyocytes from cells before differentiation can be noninvasively evaluated and diagnosed. In the Raman spectroscopy according to Experiment Example above, Raman scattering that reflects molecular vibration of molecules themselves in cells was detected.
There, the compositions of molecules in cells before differentiation were analyzed in a manner noninvasive to the cells.

Methods using Raman spectroscopy are noninvasive. Thus, the process of progress of differentiation into cardiomyocytes can be observed over time for the same cells. Therefore, methods using Raman spectroscopy are useful for studies on differentiation into cardiomyocytes. In methods using Raman spectroscopy, the quality of cells to be transplanted, for example, pluripotent stem cells can be evaluated without labeling the cells.

Next, aspects of the present invention other than the methods for evaluating cardiomyocytes, which have been presented in Experiment Examples above will be described.

### <Examples of means for accelerating maturation>

In an aspect of the present invention, artificial treatment is applied to immature cardiomyocytes to accelerate maturation. Examples of the artificial treatment include, but are not limited to, physical methods including mechanical stimulation and electrical stimulation, biological methods including introduction of a gene into cardiomyocytes, and co-culture with other cells, and chemical methods including addition of an oxidizing substrate and other compounds or other factors to a medium for cardiomyocytes. Thereafter, evaluation methods of the present invention are applied to the treated cardiomyocytes to evaluate a change in gene expression level in the cardiomyocytes, a change in protein level, and the state of progress of maturation of the myocardial function. Suitable artificial treatment is selected depending on a specific form of cardiomyocytes, that is, depending on whether the cardiomyocytes are a cell sheet, or a cell mass, or dispersed cells.

In an aspect of the present invention, cardiomyocytes are a sheet-shaped tissue fragment. In an aspect thereof, cardiomyocytes aggregate on an orientational scaffold to form a sheet-shaped tissue fragment as shown in Patent Literature 5. Cardiomyocytes can mimic a myocardial structure in a living organism due to the presence of an orientational scaffold. The cardiomyocytes forming a sheet-shaped tissue fragment are cultured over time to mature the gene expression level, the protein expression level and the myocardial function level in the cardiomyocytes. In other aspects, cardiomyocytes form a sheet-shaped tissue fragment without use of an orientational scaffold. This accelerates maturation of the cardiomyocytes.

In an aspect of the present invention, cardiomyocytes three-dimensionally aggregate to form a cell mass. In an aspect thereof, cardiomyocytes aggregate around a core to form a torus-formed cell mass as shown in Patent Literature 6. The cardiomyocytes forming a torus-formed cell mass are cultured over time to mature the cardiomyocytes. In other aspects, cardiomyocytes aggregate around a core to form a cell mass having a shape other than a torus shape. This accelerates maturation of the cardiomyocytes.

In other aspects of cardiomyocytes forming a cell mass, the cardiomyocytes form a spheroid. In an aspect thereof, cardiomyocytes form a spheroid with interstitial cells of the heart as shown in Non Patent Literature 3. In an aspect thereof, the interstitial cells of the heart are cardiac fibroblast cells and cardiac endothelial cells. Cardiomyocytes are co-cultured with these interstitial cells to mature the cardiomyocytes. In an aspect thereof, cardiomyocytes and these interstitial cells are further co-cultured with mesenchymal cells to mature the cardiomyocytes.

In other aspects of cardiomyocytes forming a cell mass, the cardiomyocytes form a cardiac organoid.

In an aspect of the present invention, cardiomyocytes are cultured under specific culture conditions before the acquisition of a Raman spectrum. The culture conditions are, for example, presence or absence of physical treatment and chemical treatment. The culture conditions also include, for example, the duration time, the quiescent time and the number of the treatments.

In an aspect of the present invention, maturation of cardiomyocytes is accelerated by mechanical stimulation. In an aspect thereof, cardiomyocytes are repeatedly expanded and contracted. As shown in Non Patent Literature 4, a mechanical stimulus is applied to cardiomyocytes using Stage Flexer (trade mark) supplied from FLEXCELL Company.

In an aspect of the present invention, maturation of cardiomyocytes is accelerated by electrical stimulation. In an aspect thereof, an electrical stimulus is applied to cardiomyocytes by an electrode as shown in Non Patent Literatures 5 and 6.

In an aspect of the present invention, maturation of cardiomyocytes is accelerated by introduction of a gene into the cardiomyocytes. An example of gene introduction is introduction of CDH2 (Non Patent Literature 7).

In an aspect of the present invention, maturation of cardiomyocytes is accelerated by culturing the cardiomyocytes in a medium containing an oxidizing substrate, for example, lipid. In an aspect thereof, palmitate is used as the oxidizing substrate as shown in Non Patent Literature 8.

In an aspect of the present invention, cardiomyocytes are matured by culturing the cardiomyocytes for a long period as shown in Non Patent Literature 9 before the acquisition of a Raman spectrum.

In an aspect of the present invention, cardiomyocytes are cultured in the presence of a drug before the acquisition of a Raman spectrum. The state of progress of maturation of the cardiomyocytes is evaluated. On the basis of the above, an effect of the drug on maturation of cardiomyocytes is evaluated.

### <Examples of Raman stereoscopic methods>

In an aspect of the present invention, a suitable Raman spectroscopic method is selected depending on a specific form of cardiomyocytes, that is, depending on whether the cardiomyocytes are a cell sheet, or a cell mass, or dispersed cells. A Raman spectroscopic method with linear illumination excitation light according to an aspect of the present invention has been described with reference to Fig. 2. Hereinafter, Raman spectroscopic methods according to other aspects of the present invention will be described.

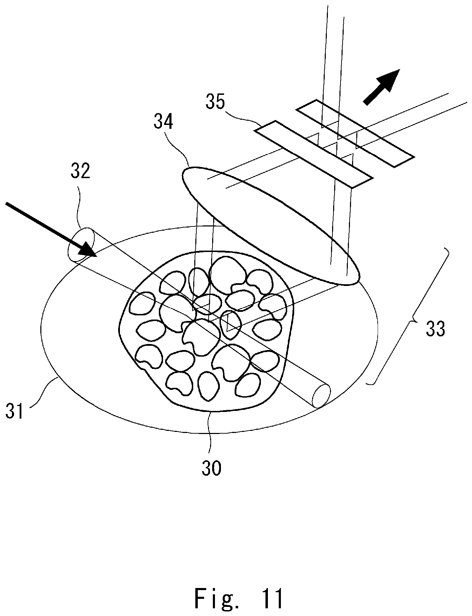

Fig. 11 shows a schematic diagram of Raman spectroscopy on a cell mass 30. The present method is so-called microscopy analysis with a lateral illumination. The cell mass 30 has a three-dimensional structure, and therefore is larger in thickness than a single-layered cell sheet and dispersed cells. An example of the cell mass 30 is a spheroid.

Excitation light 32 shown in Fig. 11 is a Bessel beam. The Bessel beam is focused on one cell or a limited number of cells in the cell mass 30. Raman-scattered light 33 is generated from the cells receiving the excitation light 32. The Bessel beam focusses to prevent expansion of Raman-scattered light generated from other cells and a culture solution 31. Therefore, little Raman-scattered light is generated behind cells to be measured by Raman spectroscopy.

A convex lens 34 fucuses the diffusing Raman-scattered light 33 as shown in Fig. 11. The focused Rama-scattered light passes through a spectroscope 35. A diffraction grating of the spectroscope 35 is installed parallel to the optical axis of the excitation light 32. The spectroscope 35 disperses the Raman-scattered light 33 by wavelengths. As above, Raman spectroscopy is implemented.

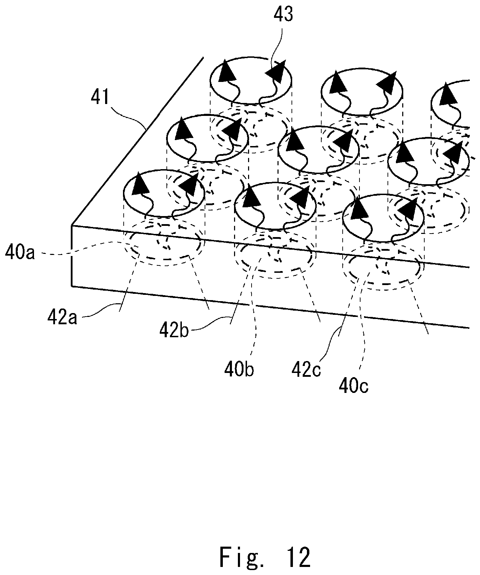

Fig. 12 shows a schematic diagram of Raman spectroscopy on a well plate 41. The present method is so-called multifocal spectroscopic analysis. The present method is suitable for evaluating, by Raman observation, a large number of samples individually conditioned with a plurality of parameters. Appropriate examples include screening of drug responses. The method is also suitable for spectroscopic analysis of a cardiomyocyte sheet having a relatively large size, for example, a well bottom surface size.

As shown in Fig. 12, cardiomyocyte sheets 40a to 40c and other cardiomyocyte sheets is disposed in the wells of a well plate, with one sheet in one well. By a multifocal Raman spectroscopic apparatus, a plurality of excitation light 42a to 42c and other excitation light each having a focal point are generated. Each cardiomyocyte sheet is irradiated with each excitation light. Conditions for inducing maturation of the cardiomyocyte sheet are different from well to well. From the cardiomyocyte sheets induced to mature under different conditions, Raman-scattered light 43 is acquired in parallel in the plate. From these Raman-scattered light 43, a Raman spectrum is acquired per well.

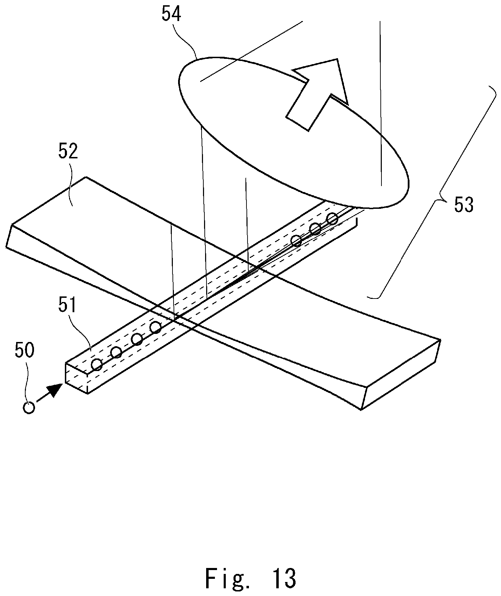

Fig. 13 is a schematic diagram of Raman spectroscopy in microchannels 51 called a flow cell. The present method is suitable for Raman spectroscopy performed on independently dispersed cells, or independently dispersed cell masses having a relatively small size. Cells 50 in the microchannels are irradiated with sheet-shaped excitation light 52. The cells 50 are independently dispersed in isolation. Raman-scattered light 53 is acquired from the cells 50 receiving the excitation light 52. A convex lens 54 fucuses the Raman-scattered light 53.

In an aspect of the method shown in Fig. 13, further, the cells 50 are sorted downstream of the microchannels 51. This increases the density of cells which have or do not have predetermined intensity of Raman-scattered light at a predetermined Raman shift in the cells 50. Such sorting improves the quality of the cells 50.

This application claims priority based on Japanese Patent Application No. 2022-005252 filed on 17 January, 2022, and the disclosure thereof is incorporated herein in its entirety.

### Reference Signs List

20 CELL SHEET
22 EXCITATION LIGHT
23 RAMAN-SCATTERED LIGHT
30 CELL MASS
31 CULTURE SOLUTION
32 EXCITATION LIGHT
33 RAMAN-SCATTERED LIGHT
34 CONVEX LENS
35 RAMAN-SCATTERED LIGHT
40a-40c CARDIOMYOCYTE SHEET
41 WELL PLATE
42a-42c EXCITATION LIGHT
43 RAMAN-SCATTERED LIGHT
50 CELL
51 MICROCHANNEL
52 EXCITATION LIGHT
53 RAMAN-SCATTERED LIGHT
54 CONVEX LENS
S11-13 STEP

## Claims

1. A method for evaluating cardiomyocytes using Raman scattering, the method comprising:
acquiring a Raman spectrum of cardiomyocytes artificially induced to differentiate from pluripotent stem cells;
acquiring an intensity of Raman-scattered light for a protein containing at least one of heme b and heme c as a prosthetic group from the Raman spectrum; and
evaluating a state of progress of maturation of the cardiomyocytes on the basis of the intensity of the Raman-scattered light.

2. The method according to claim 1, wherein
the protein containing at least one of heme b and heme c as a prosthetic group is reduced cytochrome c, and
the intensity of the Raman-scattered light, which is Raman-scattered light for the reduced cytochrome c is acquired, and a peak of the intensity is calculated on the basis of an intensity of Raman-scattered light at a Raman shift of 743 cm⁻¹ to 755 cm⁻¹ in wavenumber.

3. The method according to claim 1, wherein
the protein containing at least one of heme b and heme c as a prosthetic group is oxymyoglobin, and
an intensity of Raman-scattered light for the oxymyoglobin is acquired, and the intensity is calculated on the basis of at least an intensity of Raman-scattered light at a Raman shift of 565 cm⁻¹ to 575 cm⁻¹ in wavenumber.

4. The method according to any one of claims 1 to 3, wherein the Raman spectrum excited by light having a wavelength of 532 nm is used.

5. The method according to any one of claims 1 to 4, wherein
an intensity of Raman-scattered light for lipid is further acquired from the Raman spectrum, and
a state of progress of maturation of cardiomyocytes is evaluated on the basis of the intensity of the Raman-scattered light for lipid.

6. The method according to any one of claims 1 to 5, wherein the pluripotent stem cells are human cells.

7. The method according to claim 6, wherein the cardiomyocytes aggregate into a sheet-shaped tissue fragment.

8. The method according to claim 6, wherein the cardiomyocytes aggregate around a core to form a cell mass.

9. The method according to claim 6, wherein
the cardiomyocytes form a spheroid with human cardiac fibroblast cells, human cardiac endothelial cells and human mesenchymal cells, or
the cardiomyocytes form a spheroid with human cardiac fibroblast cells and human cardiac endothelial cells.

10. The method according to claim 6, wherein the cardiomyocytes form a cardiac organoid.

11. The method according to claim 6, further comprising, before acquiring the Raman spectrum, accelerating maturation of the cardiomyocytes by at least one of:
application of a mechanical stimulus to the cardiomyocytes;
application of an electrical stimulus to the cardiomyocytes;
introduction of a gene to the cardiomyocytes;
co-culture of the cardiomyocytes with other cells; and
addition of an oxidizing substrate, a compound and a factor to a medium in which the cardiomyocytes are cultured.

12. The method according to any one of claims 6 to 11, wherein acquiring the Raman spectrum, acquiring the intensity of the Raman-scattered light and evaluating the state of progress of maturation is repeatedly applied to the same cardiomyocytes over time.

13. The method according to any one of claims 6 to 12, further comprising, before acquiring the Raman spectrum, culturing the cardiomyocytes in the presence of a drug, wherein
the state of progress of maturation of the cardiomyocytes is evaluated to evaluate an effect of the drug on maturation of the cardiomyocytes.

14. The method according to any one of claims 1 to 13, wherein the pluripotent stem cells are one of embryonic stem cells (ESCs), induced pluripotent stem cells (iPSCs) and embryonic germ cells (EGCs), and
the embryonic stem cells (ESCs) include nuclear transfer embryonic stem cells (ntESCs).

15. The method according to any one of claims 1 to 14, wherein a peak of excitation light in the acquisition of the Raman spectrum is in a range of 400 nm to 600 nm.

16. The method according to any one of claims 1 to 15, wherein a temperature of the cardiomyocytes is decreased to 4°C or lower in the acquisition of the Raman spectrum.

17. A method for evaluating differentiation into cardiomyocytes using Raman scattering, the method comprising:
artificially inducing cells, which are pluripotent stem cells, to differentiate into cardiomyocytes;
acquiring a Raman spectrum of the cells induced to differentiate;
acquiring an intensity of Raman-scattered light for at least one of heme b and heme c from the Raman spectrum; and
evaluating a state of progress of differentiation of the cells into cardiomyocytes on the basis of the intensity of the Raman-scattered light.
